## Europäisches Patentamt

## European Patent Office

## Office européen des brevets

(19)

(11) Numéro de publication: **0 100 691**

**B1**

(12)

# FASCICULE DE BREVET EUROPÉEN

(45) Date de publication du fascicule du brevet:
**13.01.88**

(21) Numéro de dépôt: **83401239.5**

(22) Date de dépôt: **16.06.83**

(51) Int. Cl.⁴: **A 01 G 7/00,** A 01 H 17/00,
C 12 N 15/00

(54) **Procédé d'obtention in vitro de champignons endomycorhiziens à vésicules et à arbuscules.**

(30) Priorité: **21.06.82 FR 8210768**

(43) Date de publication de la demande:
**15.02.84 Bulletin 84/7**

(45) Mention de la délivrance du brevet:
**13.01.88 Bulletin 88/2**

(84) Etats contractants désignés:
**AT BE CH DE FR GB IT LI LU NL SE**

(56) Documents cité:
**EP-A-0 015 103**

**C.R. Acad. Sc. Paris, toma 292 (1981) de D.A. Tepfer
Nature, Vol. 295 (Feb. 1982) New Phytol. (1981) 88,
641-647 and 89, 87-93**

(73) Titulaire: **RHONE-POULENC AGROCHIMIE, 14-20,
rue Pierre Baizet, F-69009 Lyon (FR)**

(72) Inventeur: **Mugnier, Jacques, 63, rue du Cherche
Midi, F-75006 Paris (FR)**
Inventeur: **Jung, Gérard, rue des Grands Jardins
Leuville/s/orge, F-91310 Montlhery (FR)**
Inventeur: **Prioul, Jean-Louis, 34, Résidence du
Bois Fleuri, F-91940 Les Ulis (FR)**

(74) Mandataire: **Chrétien, François, RHONE-POULENC
AGROCHIMIE-DPI B.P. 9163, F-69263 Lyon
Cédex 09 (FR)**

**0 100 691**

**Description**

La présente invention a trait à un procédé d'obtention in vitro, de champignons endomycorhiziens à vésicules et à arbuscules (VAM).

Les récents développements de la recherche sur les mycorhizes endotrophes (endomycorhizes) ont montré que leur utilisation en agriculture et en horticulture est potentiellement très importante.

Ces champignons endophytes sont de très loin les plus fréquents et entrent en symbiose avec pratiquement toutes les plantes cultivées. Ils se caractérisent par au moins trois propriétés fondamentales.

1. leur association avec une plante est obligatoire, pour leur développement,

2. ils possèdent un large éventail d'hôtes et jusqu'à présent aucune spécificité n'a été signalée,

3. ils stimulent la croissance des plantes qu'ils infectent. L'effet favorable est un fait bien établi, et est dû à une augmentation de l'absorption des ions minéraux par la plante mycorhizée et tout spécialement des ions peu mobiles principalement les ions phosphore.

On a montré que les hyphes du champignon endomycorhizogène sont capables d'absorber, de transporter et de transférer à la plante le phosphore soluble; ces processus aboutissent à une meilleure croissance de la plante mycorhizée. (GIANINAZZI S. 1976 Physiol Vég. 14 733-741).

De plus, un certain nombre d'observations ont montré que l'infection par un champignon endomycorhizien peut protéger les racines de la plante mycorhizée contre les pathogènes du sol (BALTRUSCHAT H, 1975: Phytopathol 84, 172-188).

Mais en dépit de nombreux travaux effectués jusqu'à ce jour, (RHODES L.H., 1980, Outlook Agricult., 10, 275-281; HAYMAN D.S., 1980, Nature, 287 487-490) la principale difficulté d'application des mycorhizes endotrophes est que l'on ne sait pas les produire en grande quantité pour une application commerciale. A la connaissance de la demanderesse on n'a jamais pu les cultiver en milieu stérile sans la plante hôte. Contrairement à la plupart des micro-organismes connus symbiotiques parasites ou saprophites, les champignons VAM ne peuvent pas être cultivé in vitro. (BARRET J.T.. 1947 Phytopathol., 37, 359; MOSSE B., 1962, J. gen. microbiol., 27, 509-520; HARLEY J.L., 1965, in Ecology of soilborne plant pathogens, Baker and Snyder Eds, Univ. California Press, 218-229. HEPPER C.M., 1979, Soil Biol. Biochem., 11, 269-277; HEPPER C.M., 1981, New Phytol., 88, 641-647).

Les très nombreux essais de mycorhization réalisés à ce jour ont consisté à utiliser des inoculums préparés à partir de plantes entières cultivées en pot ou en serre.

L'inoculation s'effectue presque toujours avec les racines mycorhizées fraîchement récoltées ou parfois avec une suspension de spores.

JACKSON et al (1972, Proc. Soil. Sci. Soc. Am. 36, 64-67) utilisent des racines lyophilisées tandis que HALL (1979, Soil Biol. Biochem 11, 85-86) préconise l'emploi de boulettes de sol mélangées avec des racines infectées.

Dans tous les cas l'une des objections faites est la possibilité d'introduire des contaminants dans les sols par l'inoculation des VAM, notamment quand il est nécessaire pour des raisons phytosanitaires de procéder à une désinfection du sol avant plantation (qui a pour conséquence de détruire les VAM naturellement présentes).

La période nécessaire pour produire un inoculum est de 2 à 4 mois, ce qui est suffisant pour produire de nombreux contaminants.

Ces risques ont gêné la commercialisation des inoculums. De plus le coût de ces inoculums est au moins égal ou supérieur à celui d'engrais nécessaires pour obtenir le même rendement.

Par conséquent, l'utilisation à grande échelle, d'endomycorhizes n'est pas encore passée dans la pratique agricole, puisqu'elle se heurte à un problème majeur qui est la production d'un inoculum abondant, non contaminé, facile à stocker et à manipuler au champ.

Pour résoudre ce problème, différentes études ont été entreprises sur le comportement in vitro des champignons VAM, associé à des plantes entières ou à des racines. Si ces méthodes sont très utiles pour étudier le métabolisme des VAM, elles paraissent cependant aléatoires comme moyen de production du champignon en culture pure du fait d'un développement très lent des racines. (HEPPER C.H. 1975, in Endomycorrhizas, Sander F.E., Mosse B. and Tinker P.B. Eds, Acad Press London, 67-86; MOSSE B. and HEPPER C.M., 1975, Physiol. Pl. Pathol., 5, 215-223; HEPPER C.M., 1981, New Phytol., 88, 641-647, MACDONALD R.M., 1981, New Phytol., 89, 87-93; ST JOHN T.V. and REID C.P.P., 1981, New Phytol., 89, 81-86).

On sait par ailleurs, que la transformation génétique des racines par insertion d'un fragment d'ADN étranger (appelé T.DNA) provenant du plasmide Ri (Root inducing) d'Agrobacterium rhizogenes, permet d'établir des cultures aseptiques de racines présentant une croissance rapide et indéfinie sur milieu simple.

(TEPFER D.A. 1981. C.R. Acad. Sc. PARIS, t. 292. Série III, 153).

L'agrobacterium rhizogenes est l'agent d'une maladie, le hairyroot dont le symptome se manifeste par la prolifération d'un abondant chevelu racinaire au point d'inoculation de la bactérie.

Ainsi, on s'est aperçu que l'on pouvait résoudre le problème posé en mettant en oeuvre les facultés nouvelles conférées aux racines transformées sous l'action d'Agrobacterium rhizogenes, en inoculant des cultures de ces racines transformées par des spores d'Endomycorhizes.

L'objet de la présente invention est un procédé pour l'obtention d'endomycorhizes in vitro, notamment en fermenteur.

Ainsi, l'invention a trait à un procédé de production de champignons endomycorhiziens à arbuscules et à vésicules (VAM), comprenant la culture de racines de dicotylédones en culture aseptique à l'aide d'un milieu nutritif, suivie d'une inoculation des dites racines par des spores d'endomyccorhizes, caractérisé en ce que, in vitro, avant leur culture les racines de dicotylédones sont transformés génétiquement par infection des racines de dicotylédones par une bactérie virulente porteuse du plasmide Ri ou Ti, de manière que les gènes de ce plasmide Ri ou Ti soit inséré dans le génome des racines et que, ensuite, après élimination de la bactérie inductrie, ces racines transformées sont cultivées sur un milieu nutritif approprié ou par des moyens équivalents, de manière à obtenir des racines transformées dévelopées. La préparation de ces racines transformées, inoculées par des spores d'endomycorhizes peut être divisée en trois étapes:
- transformation de la racine
- culture de la racine transformée
- mycorhization

La transformation de la racine a lieu comme précisé précédemment par transfert dans le génome de la cellule de la racine de gènes du plasmide Ri provenant de la bactérie Agrobacterium rhizogenes.

Le milieu de culture de la bactérie Agrobacterium rhizogenes ou de la bactérie porteuse du plasmide inducteur peut être de tout type adéquat connu, tel que milieu Yeast Extract Mannitol (VINCENT, 1970, A Manual for practical study of root nodule bacteria IBP Handbook N° 15), ou milieu Sucrose, Nutrient Broth, Yeast Extract (ANAND V.K. 1977, American Journal of Botany, 64 (2) 153-158).

Les gènes du plasmide Ri peuvent être apportées de plusieurs manières, soit directement par la bactérie Agrobacterium rhizogenes, virulente porteuse du plasmide, soit indirectement par une bactérie dans laquelle on a transféré le plasmide Ri, par conjugaison ou dans laquelle on a transféré le T.DNA par transformation.

Le concept de la présente invention s'étend également au cas où l'on produit des racines de dicotylédones transformées génétiquement par insertion dans le génome, desdites racines de gènes du plasmide Ti (Tumor inducing) provenant d'Agrobacterium tumefaciens, puis que l'on inocule, lesdites racines par des spores d'endomycorhizes.

Les gènes du plasmide Ti peuvent également être apportées de plusieurs manières, soit directement par la bactérie Agrobacterium tumefaciens, virulente porteuse du plasmide, soit indirectement par une bactérie dans laquelle on a transféré le plasmide Ti, par exemple, par conjugaison ou dans laquelle on a transféré le T.DNA par transformation.

Dans le premier cas (plasmide Ri), l'agent inducteur, qui est le plasmide, peut être transféré de la souche d'Agrobacterium rhizogenes à une souche d'Agrobacterium tumefaciens, c'est le cas par exemple du transconjugant C 58 Cl (pTi 8196) (CHILTON M.D. Nature, 1982, 295, 432).

Dans le deuxième cas (plasmide Ti), l'agent inducteur, qui est le plasmide, peut être transféré de la souche d'Agrobacterium tumefaciens virulente à une souche Agrobacterium tumefaciens non virulente, c'est le cas par exemple du transconjugant K 57, (pTi C 58) (KERR A, 1977, Nature, 265, 560), ou encore à une souche de Rhizobium, c'est le cas par exemple du transjugant Rhizobium trifolii souche 5 (pTi B 63) (HOOYKAAS P.J.J., 1977, J. Gen. Microbiol. 98, 477-484), ou encore à une souche d'Escherichia coli, c'est le cas par exemple du transconjugant K 12 (pTi RP 4) (HOLSTERS M. 1978, Molec Gen. Genet. 163, 335-338), seulement dans le dernier cas cité, on ne contrôle pas à ce jour l'expression de l'agent inducteur transféré chez Escherichia coli (E. Coli).

La physiologie de ces racines transformées est différente de celles des racines non induites. Les modifications apportées sont dues à l'insertion d'un fragment d'ADN provenant du plasmide Ri ou Ti d'A. rhizogenes ou d'A. tumefaciens ou des transconjugants utilisés) (CHILTON M.D. and al. 1982, Nature, 295, 432).

A la suite de l'introduction dans le génome de la racine, des gènes étrangers portés par le plasmide Ri ou Ti, le potentiel de l'hôte est modifié.

Les racines transformées peuvent notamment être péparées par la technique décrite par TEPFER (1981), qui consiste à en induire la formation à partir d'une graine ou d'un explant tels que tubercules, fragments de tige ou de racines. On peut aussi utiliser une jeune plantule comme décrit par JAYNES (J.M. 1981, in Biology of Rhizobiaceae. Eds KENNETH L. GILLES, Acad. Press. LONDON, p. 109).

A la connaissance de la demanderesse, peu de plantes ont été testées, mais on peut citer comme plantes sur lesquelles on a induit des racines transformées, la carotte, la pomme de terre, le soja, le haricot, le trèfle, le kalenchoe, le gingembre, le liseron, l'endive toutes des plantes appartenant à la famille des dicotylédones.

Aprés introduction du T.DNA de la bactérie dans le génome, des graines ou des explants, on cherche à éliminer la bactérie inductrice qui n'a plus alors aucune utilité.

Pour éliminer la bactérie inductrice afin d'obtenir des cultures de racines transformées aseptiques, on opère par exemple en utilisant un antibiotique, inhibant la croissance de la bactérie, tel que la carbénicilline décrit par NEISH G.A., 1975, Mycologia 67), en tenant compte du fait que le système racinaire se développe plus rapidement que la bactérie, il devient alors possible d'isoler ces racines sans l'agent inducteur.

La deuxième étape de la préparation des racines transformées mycorhizées consiste après inoculation des graines ou des explants et après l'apparition des racines, à développer les racines transformées en culture aseptique sur un milieu nutritif simple sans la bactérie inductrice. La capacité des racines transformées à croître reste stable et permanente en l'absence de la bactérie inductrice.

Les milieux de culture de racines transformées peuvent se définir comme suit. Il s'agit généralement de milieu synthétique aseptique gélosé ou liquide, contenant les usuels macro et micro- éléments, un sucre, avantageusement le saccharose et une ou plusieurs vitamines et de préférence la thiamine. Ce milieu ne contient pas de composés phytohormonaux.

Pour le cas des milieux liquides, le pH est avantageusement régulé. Le milieu peut aussi être avantageusement renouvelé au cours de la culture.

Les racines transformées peuvent être mises en culture sur milieu minéral de Monnier (Rev. Cyt. Biol. vég. 39, 1976, p. 1), saccharose, vitamines de Morel (GOLDMANN A. Ann. Sc. Nat. Bot. PARIS, 11, 1970, p. 223).

On peut utiliser le milieu de MURASHIGE T. et SKOOG F. (Physiol. Plant., 15, 473-497, 1962).

Pour certaines plantes telles que le liseron, des milieux adéquats ont été décrits, comme le milieu de BONNER, modifié TORREY J.G. (1958, Plant physiol. 33, 258-263).

La culture peut être réalisée industriellement et de façon tout à fait inattendue, pour des racines, en fermenteur.

Lorsque l'on opère en fermenteur, la croissance est très rapide mais le milieu de culture doit être correctement aéré afin d'éviter l'anoxie des racines.

La culture en fermenteur est avantageusement réalisée à température ambiante (20 à 30°C).

Les racines transformées, contrairement à la plupart des essais réalisés sur culture de racines non transformées, ne montrent aucune auxotrophie vis-à-vis des composés phytohormonaux, forment de nombreuses ramifications secondaires et ont un potentiel de croissance élevé. Il est en effet nécessaire d'utiliser des composés hormonaux type auxine pour contrôler la formation de racines latérales des racines non transformées.

Les racines transformées développées en culture aseptique constituent donc un partenaire symbiotique privilégié pour le champignon endomycorhizien dont on sait notamment que le nombre de points d'infection est augmenté par l'apparition de nombreuses racines secondaires (SMITH S.E. and WALKER N.A., 1981, 89, 225-240, New Phytol).

Les individus régénérés à partir d'une racine transformée constituent un clône de racines ayant subi un changement physiologique permanent et stable, et l'élément transformant, constitué par l'insertion de gênes étrangers, est répliqué au cours des mitoses cellulaires.

Tout clône de racines n'est pas nécessairement le plus adapté à la culture et à la mycorhization, vu que l'on considère actuellement que le T-DNA est capable de s'insérer dans des parties différentes du génome des cellules végétales, et de donner ainsi des clônes de racines différents.

A la connaissance de la demanderesse, on ignore à la fois les mécanismes d'insertion et les sites de cette insertion, mais on sait que chaque clône obtenu sera potentiellement plus ou moins favorable à la réalisation d'un système de culture de racines transformées et mycorhizées.

On doit aussi sélectionner le système végétal ayant les capacités intrinsèques de croissance les plus favorables.

Un système de culture de racines transformées génétiquement par insertion de DNA étranger, constitue un partenaire symbiotique bien plus favorable qu'un système de racines non transformées pour le développement des Endomycorhizes. Ces avantages sont résumés ci-dessous:

1. Le système de culture de racines transformées est simple et ne fait intervenir pour sa réalisation que l'effet inducteur de la bactérie Agrobacterium rhizogenes, ou Agrobacterium tumefaciens ou des bactéries porteuses du plasmide inducteur,

2. Le développement des racines transformées est possible sur un milieu de culture simple, en conditon aseptique, et ne montre aucune auxotrophie vis-à-vis de composés hormonaux,

3. Les points d'entrée du champignon VAM sont plus nombreux sur une racine transformée que sur une racine non transformée. On sait que les points d'entrée sur la racine sont limités à des zones bien définies et que les jeunes régions de la racine vers l'apex sont 10 fois plus favorables à l'infection que le reste de la racine. Une des caractéristiques des racines transformées est la formation en culture de nombreuses racines secondaires; la probabilité d'apparition de nouveaux points d'entrée secondaires est donc augmentée dans le cas des racines transformées.

4. Le potentiel de développement élevé et indéfini des racines transformées permet d'obtenir en culture une production en masse de racines mycorhizées.

5. Les potentialités nouvelles de racines transformées par introduction de gènes procaryotiques, favorables à l'infection et au développement d'un champignon endomycorhizien sont permanentes et stables.

La troisième étape du procédé de préparation selon l'invention de racines transformées inoculées par des spores d'endomycorhizes à vésicules et à arbuscules consiste à mycorhizer les racines transformées à l'aide des spores d'endomycorhizes.

Cette étape peut être divisée en quatre séquences:
- isolation des spores
- stérilisation des spores
- germination des spores
- infection de la racine

Les spores utilisables pour la mycorhization sont celles que l'on peut isoler facilement à partir des sporocarpes d'endomycorhizes prélevées sur les racines de plantes mycorhizées. On préfère les spores présentant une facilité d'utilisation, c'est-à-dire de tailles importantes (0,1 à 1 mm facilement stérilisables). Sont notamment utilisés, les spores de Glomus mossae (Abbot Laboratories Chemical and agricultural products division, 1400 Sheridan Road N. Chicago Illinois 60064 USA) et de Gigaspora margarita (Abbot Laboratories).

L'inoculation des racines doit être réalisée avec des spores d'Endomycorhizes stériles.

Pour obtenir des spores d'Endomycorhizes stériles, on peut utiliser tout système capable d'éliminer les agents contaminants et ne détruisant pas les spores. Pour cela, on peut utiliser le mélange décrit par MOSSE B. chloramine T et streptomycine (1959, Trans. Brit. mycol. Soc. 42, 273-286).

Après stérilisation, les spores sont rincées à l'eau puis mises à germer.

La germination de ces spores n'est pas évidente, l'inhibition de la germination est par contre facilement levée par la présence de microorganismes et notamment de champignons tels que Sporotrix shenkii ou d'un Actinomycète déposé au C.B.S. (Centraalbureau voor schrimmelcultures Oosterstraat 1 Po Box 273 BAARN (Nederland) sous le n° 355,83.

D'une manière pratique, on cultive sur un milieu approprié le microorganisme levant l'inhibition puis on l'inclut dans un gel d'agar, on coule par dessus cette première couche d'agar une deuxième couche d'agar stérile. On dépose sur cette deuxième couche les spores d'endomycorhizes stériles, la germination apparaît rapidement.

Le microorganisme levant l'inhibition ne doit pas envahir la couche de gélose supérieure afin de ne pas contaminer les spores d'Endomycorhizes. Il semblerait qu'il agit par l'intermédiaire d'une ou de plusieurs substances chimiques qui traverseraient la gélose et permettraient la germination des spores.

Lorsque l'on dispose de spores prégermées et d'un système racinaire provenant d'une culture réalisée par exemple en fermenteur il faut procéder à l'infection des racines. Cette infection se fait sur un milieu de culture tel que gélosé ou liquide ne contenant pas d'éléments toxiques pour l'infection tel que le manganèse ou le zinc.

Un dogme depuis longtemps établi consiste à reconnaître que des concentrations élevées en phosphore inhibent l'infection des racines de plantes entières (RHODES 1980 Outlook Agriculture, 10, 275-281). Contrairement à ce préjugé technique, le phosphore même à des concentrations élevées, par exemple 170 ppm de $KH_2Po_4$, n'inhibe pas le processus de l'infection de racines transformées.

L'azote minéral présent dans les milieux de culture précédemment décrit inhibe totalement les processus de l'infection.

Certains éléments au contraire favorisent l'infection des racines. Le calcium est favorable aux processus de l'infection ainsi que certains complexants. L'EDTA est favorable lorsqu'il est apporté à de faibles concentrations. L'EDTA peut être remplacé par d'autres complexants tels que la tourbe.

L'absence de poils absorbants favorisent aussi l'infection des racines transformées.

Le pH du milieu où a lieu l'infection est avantageusement situé entre 6 et 8.

La composition du milieu compatible avec l'infection peut être très variable car les rapports initiaux en éléments tels que carbone, azote, calcium, phosphore sont modifiés ou immobilisés par la racine transformée en croissance.

La racine transformée peut se développer vers un milieu d'infection placé dans un compartiment isolé du milieu nutritif de la racine, ce milieu d'infection étant constitué uniquement d'agar et d'eau.

Mais la présente invention sera plus aisément comprise à l'aide des exemples suivants, donnés à titre indicatif mais nullement limitatif:

**Exemple 1**

a) Agrobacterium tumefaciens, GRAM négatif, appartient à la famille des Rhizobiacées; La souche utilisée est la souche C 58 (souche de collection PHABAGEN COLLECTION, Department of Molecular Cell Biology section Microbiology. Transitorium III Padualaan 8. De Uithof. 3584 CH Utrecht the Netherlands).

b) L'Agrobacterium rhizogenes, GRAM négatif appartient à la famille des Rhizobiacées. La souche utilisée est la souche 8196. (Souche collection NCPPB, National, Collection of plant Pathogenic Bacteria, Plant Pathology Laboratory, Ministry of Agriculture Fisheries and Food, Hatching Green, Harfenden, Herts, ENGLAND).

Les deux souches sont cultivées en milieu liquide YEM.

L'induction racinaire. Des tubercules de pomme de terre ou des carottes sont stérilisées par immersion dans une solution de chlorure mercurique (0,1 %) ou d'hypochlorite de sodium (1,05 %) pendant 5 mn, puis rincées avec de l'eau stérile (10 fois). On découpe stérilement à l'emporte-pièce des disques de 10 mm de diamètre sur 4 mm de hauteur que l'on dispose sur un milieu gélosé (Agar DIFCO 1 %).

Les disques sont inoculés avec 0,1 ml de culture bactérienne (à $10^9$/ml). Environ deux semaines après l'inoculation, des racines sont apparues sur la plupart des explantats inoculés avec la souche 8196, et sur quelques explantats avec la souche C 58. Des segments de racines sont séparés du disque de pomme de terre ou de carotte, rincés par plusieurs trempages dans l'eau stérile et placés sur le milieu MONNIER-MOREL auquel on a incorporé de la carbénicilline (600 mg/l). Les racines qui continuent à se développer sur le milieu forment de très nombreuses racines secondaires. Celles-ci sont à nouveau rincées avec de l'eau stérile et transférées sur le milieu nutritif sans l'antibiotique.

Dés que les racines se sont développées sur le milieu gelosé ou liquide, les cultures sont repiquées à intervalles d'environ 2 semaines.

Composition du milieu de culture. Le milieu est composé du milieu minéral de MONNIER, d'une solution de vitamines de MOREL et de saccharose (3 %), le pH est ajusté à 6,0.

MILIEU MINERAL DE MONNIER

5

| Macro-éléments | | | Micro-éléments | |
|---|---|---|---|---|
| Sels | mM/l | mg/l | Sels | mg/l |
| $NH_4NO_3$ | 1650 | 20,8 | $H_3BO_3$ | 8,2 |
| $KNO_3$ | 1900 | 18,8 | $MnSO_4,4H_2O$ | 22,3 |
| $CaCl_2,2H_2O$ | 440 | 3,0 | $ZnSO_4,4H_2O$ | 8,6 |
| $MgSO_4,7H_2O$ | 370 | 1,5 | $KI$ | 0,83 |
| $KH_2PO_4$ | 170 | 1,2 | $NaMoO_4,2H_2O$ | 0,25 |
| $Na_2EDTA$ | 37,3 | 0,1 | $CuSO_4,5H_2O$ | 0,025 |
| $FeSO_4,7H_2O$ | 27,8 | 0,1 | $CuCl_2,H_2O$ | 0,025 |

mM = millimole

**Vitamines de MOREL**

Pantothénate de calcium: 1 mg/l; inositol: 100 mg/l; biotine: 0,01 mg/l; acide nicotinique: 1 mg/l; pyridoxine: 1 mg/l et thiamine: 1 mg/l.

On a utilisé pour la culture de racines transformées de liseron le milieu BONNER modifié THOREY.

en mg/l Ca $(NO_3)_2$ $4H_2O$ 242 - $MgSO_4$ $7H_2O$ 42 mg - $KNO_3$ 85 - KCl 61-$KH_2PO_4$ 20, $FeCl_3$ 1,5 - Thiamine HCL 0,1 - acide nicotinique 0,5 - Zn $SO_4$ 1,5 - $MnSO_4$ : 4,5-$Na_2MOO_4$ $2H_2O$ 0,25 - $H_3BO_3$ 1,5 - $CuSO_4$ 0,004 et sucrose 40 000 - pH 5,0.

Les milieux permettant soit la culture des racines transformées de pommes de terre, carotte soit de liseron ne permettent pas l'infection par l'endomycorhize.

**Exemple 2**

Culture des racines transformées de liseron en fermenteur

La culture des racines transformées en milieu liquide a été effectuée en fermenteur Biolafitte autoclavable de 2 litres et 30 litres type culture cellulaire de la façon suivante:
- milieu: milieu de MONNIER - MOREL ajusté à pH 5,8
- remplissage: 750 ml pour fermenteur de 2 l et 20 l pour celui de 30 l
- stérilisation de l'unité de fermentation à l'autoclave, durant 30 mn à 115°C; pH après stérilisation de 5,3.
- après refroidissement, le milieu est ensemencé par une préculture en boîte de Pétri de racines transformées de Convolvulus (liseron). Le milieu est agité à l'aide d'une turbine type culture cellulaire tournant à 50 t/mn et aéré par un débit d'air de 80 l/h sous pression de 0,1 $10^5$ Pa; la température de fermentation est régulée à 25°C.

On peut procéder de façon similaire en supprimant la turbine, l'aération étant assurée par le seul bullage de l'air.

Dans les deux cas, le volume du fermenteur est colonisé par les racines transformées en 2 à 3 semaines.

La production exprimée en matière sèche est alors de 13,4 g/l pour 17 jours.

**Exemple 3**

Infection des racines transformées de liseron

L'Endogonacée utilisée est une souche de Glomus mosseae provenant du laboratoire de B. MOSSE (Rothmamsted) (Department of soil microbiology - Rothmamsted experimental station. HARPENDEN HERTFORDSHIRE - ENGLAND AL 5 2JQ). Des souches de Glomus sont disponibles à ABBOT LABORATORIES CHEMICAL AND AGRINULTURAL PRODUCTS DIVISION. 1400 SHERIDAN ROAD N. CHICAGO ILLINOIS 60064 U.S.A.

**1. production des sporocarpes**

La composition du substrat de culture pour la plante hôte, pour obtenir un nombre important de sporocarpes, est la suivante: vermiculite, perlite et un sol de Fontainebleau, pauvre en phosphore, dans les proportions 5: 5: 1. Le pH du mélange est de 6,5. Après deux périodes de végétation (6 mois), le nombre de sporocarpes est d'environ 10.000/litre de substrat.

**2. isolement des spores**

Des oignons mycorhizés avec Glomus mosseae et bien d'eveloppés sont dépotés. Sous la loupe binoculaire,

6

les sporocarpes mesurant 0,1 à 1 mm de diamètre sont prélevés directement à l'aide d'une pipette et placés dans une boîte de Pétri sur papier filtre humide.

Les spores formées à l'intérieur des sporocarpes sont ensuite extraites avec une aiguille, chaque sporocarpe fournit 1 à 100 spores environ. Les spores sont conservées à 4°C.

3. **Stérilisation des spores**: On utilise le mélange stérilisant composé de streptomycine, de dichloramine T (2 %) et de quelques gouttes de Tween 80.

Pour la stérilisation des spores, on procède en deux étapes. Dans un premier temps on place un filtre grillagé (Ø < 50 μm) dans une colonne millipore et on fait passer 500 ml du mélange stérilisant sur les spores.

Dans un deuxième temps, on remplace le filtre grillagé par un papier MILLIPORE (référence AW 03 04 700) dans la colonne Millipore montée sur une fiole à vide.

On fait passer 500 ml du mélange stérilisant sur les spores en suspension dans le haut de la colonne en maintenant le niveau du liquide à environ 2 cm au-dessus de papier Millipore, en agitant constamment. Après ce traitement, les spores sont rincées six fois à l'eau stérile. Au dernier rinçage, le papier Millipore est asséché par un pompage plus long.

4. **germination des spores**

On cultive le microorganisme inducteur de la germination (actinomycète déposé à la C.B.S. sous le no 355,83) dans un milieu liquide et agité contenant les éléments suivants: glucose 10 g/l, extrait de levure 2 g/l et eau en quantité suffisante pour 1 l.

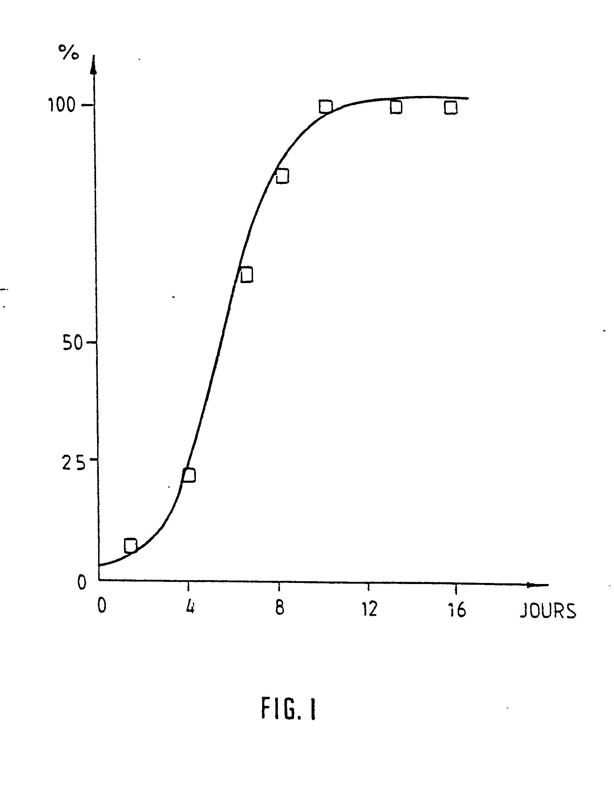

On obtient des "boulettes" (terme anglo-saxon "pellet") de ce microorganisme qui sont lavées et incluses dans de la gélose Agar Difco à 0,5 %. Sur cette gélose, on coule une deuxième couche d'agar et on dépose sur cette couche les spores stériles de l'Endomycorhize. En 10 à 15 jours, on a approximativement 100 % de germination (figure 1).

La figure 1 représente le pourcentage de germination des spores placées sur un milieu gelosé Agar Difco 0,7 % en présence de actinomycète no 355,83 en fonction du temps exprimé en jours (le lot témoin placé sur le même milieu gélosé, mais sans bactérie n'a pas donné de spores germées). Seules les spores ou les globules lipidiques qui sont bien distincts ont germé.

On considère que la spore a réellement germé lorsque le processus de germination est le suivant:

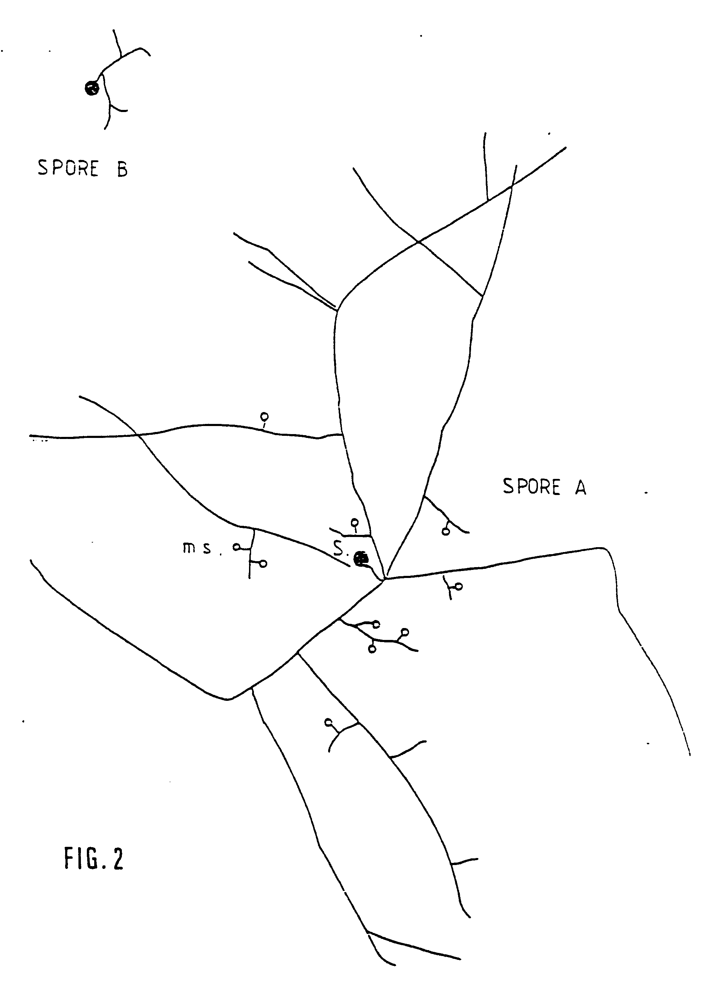

Un filament mycélien émerge de ce qui subsiste de l'ancienne hyphe d'attachement, puis se ramifie et apparaissent des micro- spores végétatives sur les hyphes. Le schéma de ce processus est illustré sur la figure 2.

La figure 2 montre:
- une spore A ayant germé en présence d'un actinomycète (m.s = microspore végétative, h = hyphe, s = spore).
- une spore B placée sur milieu gelosé sans actinomycète.

Le microorganisme inducteur de la germination ne se développe pas dans le système décrit et n'envahit donc pas la couche de gélose sur laquelle sont déposées les spores mais reste capable de produire une ou des substances inconnues qui lèvent l'inhibition de la germination.

5. **Infection de la racine**

Les spores prégermées sont placées à proximité d'une racine de <u>Convolvulus sepium</u>, trés peu pourvue en poils absorbants, placée sur un milieu pauvre en azote et en élements toxiques pour la croissance du mycélium tels que le zinc, le manganèse, ou l'azote minéral lorsqu'il est apporté en grande quantité et pourvu d'éléments stimulants tels que calcium, EDTA... L'infection de la liseron est très rapide, on observe les premières structures infectieuses trois jours après le dépôt de la spore.

Les premières structures mycéliennes caractéristiques d'une infection VA mycorhizienne après le dépôt de la spore sont constituées par des structures en éventail ("fan-like") qui évoluent après environ 10 jours vers la formation d'arbuscules dans les cellules de la racine transformée.

6. **Méthode de coloration des structures en éventail et en arbuscules**

Des fragments de racines transformées de 2 cm de longueur sont prélevés et éclaircis par une solution de KOH à 20 p. 100 pendant 1 heure à l'étuve à 90°C. Un blanchissage total des échantillons est obtenu dans une solution aqueuse à 5 p. 100 de $H_2O_2$ à 30 volumes. Les échantillons sont colorés soit par une solution à 0,05 p. 100 de bleu trypan dans le lactophénol d'Amann pendant 5 mn à chaud, soit dans une solution d'acide fuschique à 0,1 p. 100 dans un mélange eau-acide acétique (1v/10v) pendant 2 mn. La deuxième méthode a tendance à altérer sensiblement les structures fongiques mais permet par contre une meilleure différenciation champignon-cellules racinaires. Après coloration, les échantillons sont lavés et montés au lactophénol.

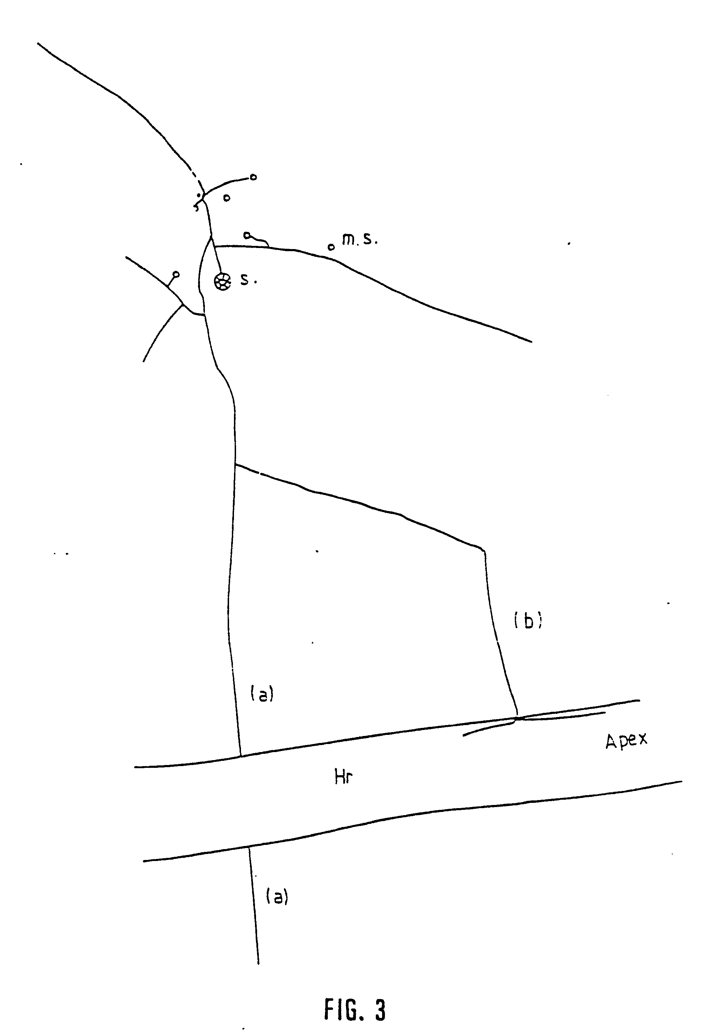

La figure 3 montre un filament mycélien ramifié en deux branches, l'une des branches (a) passe sous la racine transformée, l'autre branche (b) infecte la racine.

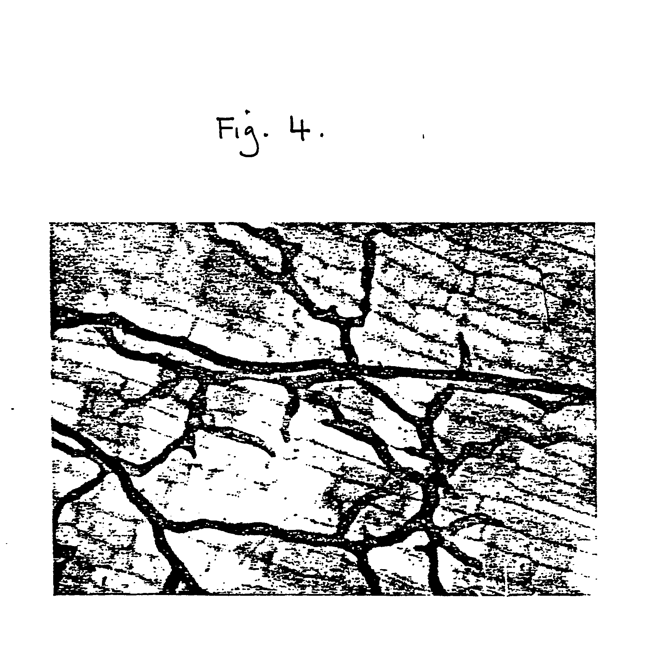

La figure 4 qui est une photo prise au microscope photonique montre la première phase de l'infection aprés 3 jours d'une racine tranformée de <u>Convolvulus sepium</u> avec les structures caractéristiques du mycélium en éventail, cloisonnée.

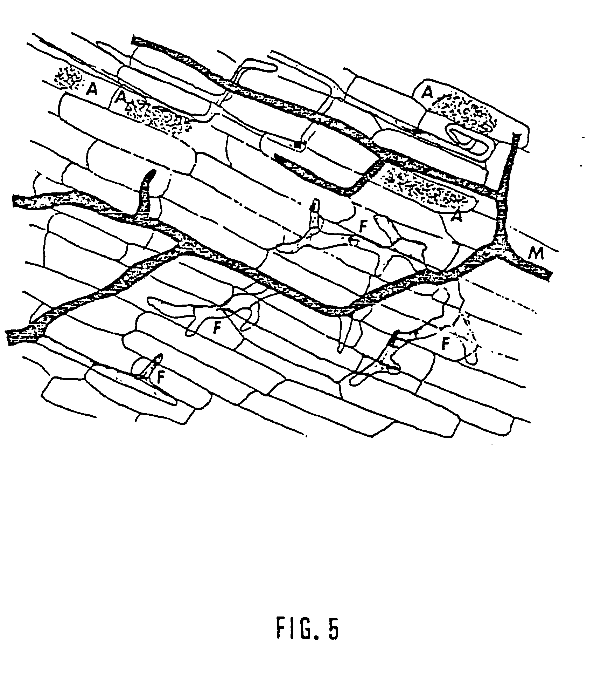

La figure 5 qui est un dessin représente la deuxiéme phase de l'infection aprés 10 jours avec la formation d'arbuscules.

M représente le mycélium.

F représente les structures en éventail (fan-like).

A représente les arbuscules.

**Revendications**

1. Procédé de production de champignons endomyccorhiziens à arbuscules et vésicules (VAM), comprenant la culture de racines de dicotylédones en culture aseptique à l'aide d'un milieu nutritif, suivie d'une inoculation des dites racines par des spores d'endomyccorhizes, caractérisé en ce que, in vitro, avant leur culture les racines de dicotylédones sont transformés génétiquement par infection des racines de dicotylédones par une bactérie virulente porteuse du plasmide Ri ou Ti, de manière que les gènes de ce plasmide Ri ou Ti soient insérés dans le génome des racines et que, ensuite, après élimination de la bactérie inductrice, ces racines transformées sont cultivées sur un milieu nutritif approprié ou par des moyens équivalents, de manière à obtenir des racines transformées développées.

2. Procédé selon la revendication 1, caractérisé en ce que la bactérie utilisée est Agrobacterium rhizogènes porteuse du plasmide Ri ou Agrobacterium tumefaciens porteuse du plasmide Ti.

3. Procédé selon la revendication 1, caractérisé en ce que la bactérie utilisée est une bactérie, dans laquelle on a préalablement transféré le plasmide Ri ou Ti.

4. Procédé selon l'une des revendications 1 à 3, caractérisé par le fait que l'endomycorhize appartient à la famille des endogonacées.

5. Procédé selon l'une des revendications 1 à 4, caractérisé par le fait que l'endomycorhize est un Glomus mosseae.

6. Procédé selon l'une des revendications 1 à 5 caractérisé en ce que les racines transformées sont obtenues à partir d'une graine de dicotylédone.

7. Procédé selon l'une des revendication 1 à 5, caractérisé en ce que les racines transformées sont obtenues à partir d'un explant de dicotylédone.

8. Procédé selon l'une des revendications 1 á 7, caractérisé par le fait que les racines transformées sont cultivées aseptiquement sur un milieu simple, sans composés hormonaux, comprenant un surce, avantageusement le saccharose, des macro-éléments et oligo-éléments et au moins une vitamine et de préférence le thiamine.

9. Procédé selon l'une des revendications 1 à 8, caractérisé par le fait que les racines transformées sont cultivées sur milieu gélosé.

10. Procédé selon l'une des revendications 1 à 8, caractérisé par le fait que les racines transformées sont cultivées dans un milieu liquide.

11. Procédé selon l'une des revendications 1 à 8 et 10, caractérisé par le fait que les racines transformées sont cultivées en fermenteur en milieu aéré et régulé.

12. Procédé selon l'une des revendications précédentes, caractérisé par le fait que l'inhibition de germination des spores des VAM est levée par un microorganisme.

13. Procédé selon la revendication 12 caractérisé par le fait que le microorganisme leveur de l'inhibition est choisi parmi Sporotrix shenkii et un actinomycète.

14. Procédé selon l'une quelconque des revendications précédentes caractérisé par le fait que l'infection de la racine par les spores d'endomycorhize est réalisée sur un milieu à pH compris entre 6 et 8 sans composé toxique pour l'infection tel que le manganèse, le zinc, et pauvre en azote minéral, mais en présence de calcium et d'un agent complexant tel que l'EDTA.


**Patentansprüche**

1. Verfahren zur Produktion von vesikulär-arbuskulären Endomycorrhiza-Pilzen (VAM)
durch
Kultivierung von Wurzeln von Dicotyledonen in aseptischer Kultur mit Hilfe eines Nährmediums
und
Beimpfung der Wurzeln mit Endomycorrhiza-Sporen,
dadurch gekennzeichnet, daß
die Wurzeln der Dicotyledonen vor der Kultivierung in vitro durch Infizierung mit einem virulenten Bakterium, welches das Plasmid Ri oder Ti aufweist, derart genetisch transformiert werden, daß die Gene des Plasmids Ri oder Ti in das Genom der Wurzeln eingefügt werden, und anschließend die transformierten Wurzeln nach Abtrennung des zur Induktion herangezogenen Bakteriums auf einem geeigneten Nährmedium oder mit äquivalenten Mitteln so kultiviert werden, daß entwickelte transformierte Wurzeln erhalten werden.

2. Verfahren nach Anspruch 1, dadurch gekennzeichnet, daß als Bakterium Agrobacterium rhizogenes, welches das Plasmid Ri aufweist, oder Agrobacterium tumefaciens, welches das Plasmid Ti aufweist, eingesetzt wird.

3. Verfahren nach Anspruch 1, dadurch gekennzeichnet, daß ein Bakterium eingesetzt wird, in das zuvor das Plasmid Ri oder Ti übertragen wurde.

4. Verfahren nach einem der Ansprüche 1 bis 3, dadurch gekennzeichnet, daß die Endomycorrhiza der Familie der Endogonaceen angehört.

5. Verfahren nach einem der Ansprüche 1 bis 4, dadurch gekennzeichnet, daß die Endomycorrhiza Glomus mosseae ist.

6. Verfahren nach einem der Ansprüche 1 bis 5, dadurch gekennzeichnet, daß die transformierten Wurzeln ausgehend von einem Dicotyledonen-Samenkorn hergestellt werden.

7. Verfahren nach einem der Ansprüche 1 bis 5, dadurch gekennzeichnet, daß die transformierten Wurzeln ausgehend von einem Dicotyledonen-Explantat hergestellt werden.

8. Verfahren nach einem der Ansprüche 1 bis 7, dadurch gekennzeichnet, daß die transformierten Wurzeln unter aseptischen Bedingungen auf einem einfachen Medium ohne Gehalt an Hormonverbindungen kultiviert werden, das einen Zucker, vorteilhaft Saccharose, Makroelemente und Spurenelemente und mindestens ein Vitamin und vorzugsweise Thiamin enthält.

9. Verfahren nach einem der Ansprüche 1 bis 8, dadurch gekennzeichnet, daß die transformierten Wurzeln auf einem Gelosemedium kultiviert werden.

10. Verfahren nach einem der Ansprüche 1 bis 8, dadurch gekennzeichnet, daß die transformierten Wurzeln in einem flüssigen Medium kultiviert werden.

11. Verfahren nach einem der Ansprüche 1 bis 8 und 10, dadurch gekennzeichnet, daß die transformierten Wurzeln in einem Fermenter in einem belüfteten und geregelten Medium kultiviert werden.

12. Verfahren nach einem der vorhergehenden Ansprüche, dadurch gekennzeichnet, daß die Inhibierung der Sporenkeimung durch einen Mikroorganismus aufgehoben wird.

13. Verfahren nach Anspruch 12, dadurch gekennzeichnet, daß der zur Aufhebung der Sporenkeimung eingesetzte Mikroorganismus unter Sporotrix shenkii und einem Actinomyceten ausgewählt wird.

14. Verfahren nach einem der vorhergehenden Ansprüche, dadurch gekennzeichnet, daß die Infizierung der Wurzeln durch die Endomycorrhiza-Sporen auf einem Medium mit einem pH-Wert zwischen 6 und 8 vorgenommen wird, das keine für die Infektion toxische Verbindung, wie Mangan und Zink, enthält und arm an mineralischem Stickstoff ist, jedoch in Gegenwart von Calcium und einem Komplexbildner wie EDTA.

## Claims

1. A method of producing endomycorrhizian fungi with arbuscles and vesicles (VAM) comprising inducing and developing dicotyledone roots in an aseptic culture on a nutrient medium, then inoculating said roots with endomyccorhiza spores characterized in that, in vitro, before their culture, the dicotyledone roots are genetically converted by infection of dicotyledone roots with a Ri or Ti plasmid bearing virulent bacterium, so that genes of this Ti or Ri plasmid should be inserted in the root genom and that, thereafter, after removing the inducing bacterium, these converted roots are cultivated on an appropriate nutrient medium or with equivalent means, to get the developped transformed roots.

2. The method of claim 1, characterized in that said bacterium is the Ri plasmid bearing virulent bacterium Agrobacterium rhizogenes or Ti plasmid bearing virulent bacterium Agrobacterium tumefaciens.

3. The method of claim 1, characterized in that said bacterium is a bacterium to which the root inducing plasmid has been previously transferred.

4. The method of any of claims 1 to 3, characterized in that endomycorrhiza belongs to endogonacae is used.

5. The method of any of claims 1 to 4, characterized in that the endomycorrhiza is a Glomus mosseae.

6. The method of any of claims 1 to 5, characterized in that the converted roots are obtained from a dicotyledone seed.

7. The method of any of claims 1 to 5, characterized in that the converted roots are obtained from a dicotyledone explant.

8. The method of any of claims 1 to 5, characterized in that the converted roots are cultivated aseptically on a simple medium without any hormone compounds, comprising a sugar, advantageously saccharose, macro-elements and oligo-elements and at least one vitamin, preferably thiamin.

9. The method of any of claims 1 to 8, characterised in that the converted roots are cultivated on a gelose medium.

10. The method of any of claims 1 to 8, characterized in that the converted roots are cultivated in a liquid medium.

11. The method of any of claims 1 to 8 and 10, characterized in that the converted roots are cultivated in a fermenter, in an aerated and controlled medium.

12. Process of any of the preceeding claims, characterized in that the inhibition of germination of VAM spores is removed through the presence of a microorganism.

13. Process of claim 12, characterized in that the inhibition removing microorganism is chosen in the group consisting of Sporotrix shenkii and an actinomycete.

14. The method of any of the preceding claims, characterized in that the root inoculation with endomycorrhiza spores is carried out on a medium having a pH between 6 and 8, which is free of compounds toxic for the infection, such as traces of zinc, copper and iron, and low in mineral nitrogen, but in presence of calcium and a complexing agent such as EDTA.

FIG. I

SPORE B

SPORE A

m s.

S.

FIG. 2

0 100 691

m.s.

s.

(b)

(a)

Apex

Hr

(a)

FIG. 3

5

Fig. 4.

FIG. 5